# EUROPEAN PATENT APPLICATION

(11) **EP 1 908 471 A1**
(43) Date of publication of application: **09.04.2008**
(21) Application number: 07117835.4
(22) Date of filing: 03.10.2007
(51) Int. Cl.: A61K 31/44, C07D 211/78, A61P 9/12

(54) **Tetrahydropyridines as renin inhibitors**

(30) Priority: 04.10.2006 EP 06121771
(71) Applicant: Speedel Experimenta AG, 4123 Allschwil (CH)
(72) Inventor: Herold, Peter, 4123, Allschwil (CH); Mah, Robert, 4123, Allschwil (CH); Marti, Christiane, 4123, Allschwil (CH)
(74) Representative: Maué, Paul Georg

(57) **Abstract**

The application relates to novel substituted tetrahydropyridines of the general formulae (I) and (II) 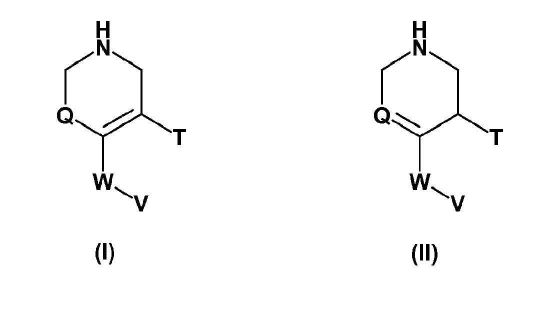 and the salts thereof, preferably the pharmaceutically acceptable salts thereof, in which Q, T, V and W have the meanings defined in the description, to a process for the preparation thereof and to the use of these compounds as medicines, especially as renin inhibitors.

## Description

### FIELD OF THE INVENTION

The present invention relates to novel substituted tetrahydropyridines, processes for their preparation and the use of the compounds as medicines, especially as renin inhibitors.

### PRIOR ART

Tetrahydropyridine derivatives for use as medicines are disclosed for example in WO 2004/002957. However, in terms in particular of renin inhibition, there continues to be a need for highly potent active ingredients. The priority in this connection is improving the pharmacokinetic properties. These properties, which are directed at better bioavailability, are for example absorption, metabolic stability, solubility or lipophilicity.

### DETAILED DESCRIPTION OF THE INVENTION

The invention therefore relates firstly to substituted tetrahydropyridines of the general formulae (I) and (II) 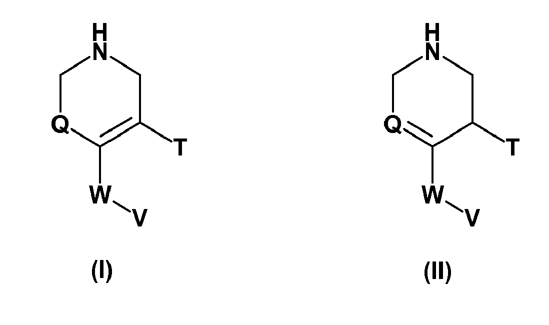 wherein
**M** (as part of T) is aryl or heterocyclyl, especially benzoimidazolyl, benzo[1,3]dioxolyl, benzofuranyl, benzooxazolyl, benzothiazolyl, benzo[b]thienyl, 2H-chromenyl, 3,4-dihydro-2H-benzo[1,4]oxazinyl, dihydro-3H-benzo[1,4]oxazinyl, dihydro-2H-benzo[1,4]thiazinyl, 1 a,7b-dihydro-1 H-cyclopropa[c]chromenyl, 2,3-dihydro-1 H-indolyl, dihydro-1H-pyrido[2,3-b][1,4]oxazinyl, imidazo[1,2-a]pyridyl, imidazo[1,5-a]pyridyl, indazolyl, indolyl, isobenzofuranyl, isoquinolyl, [1,5]naphthyridyl, phenyl, phthalazinyl, pyridyl, pyrimidinyl, 1 H-pyrrolo[2,3-b]pyridyl, 1 H-pyrrolo[2,3-c]pyridyl, 1H-pyrrolo[3,2-b]pyridyl, quinazolinyl, quinolyl, quinoxalinyl, tetrahydroimidazo[1,2-a]pyridyl, 1,1a,2,7b-tetrahydro-cyclopropa[c]chromenyl, tetrahydroimidazo[1,5-a]pyridyl, tetrahydroisoquinolyl, tetrahydroquinolyl, tetrahydroquinoxalinyl, [1,2,3]triazolo[1,5-a]pyridyl or [1,2,4]triazolo[4,3-a]pyridyl, each of which is optionally substituted by 1-4 acyl-C₁₋₈-alkoxy-C₁₋₈-alkoxy, acyl-C₁₋₈-alkoxy-C₁₋₈-alkyl, (N-acyl)-C₁₋₈-alkoxy-C₁₋₈-alkylamino, C₁₋₈-alkanoyl, C₁₋₈-alkoxy, C₁₋₈-alkoxy-C₁₋₈-alkanoyl, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, (N-C₁₋₈-alkoxy)-C₁₋₈-alkylaminocarbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkyl-carbamoyl, C₁₋₈-alkoxy-C₁₋₈-alkyl-carbonylamino, C₁₋₈-alkoxy-C₁₋₈-alkyl-heterocydyl, C₁₋₈-alkoxy-carbonyl, C₁₋₈-alkoxycarbonyl-C₁₋₈-alkoxy, C₁₋₈-alkoxycarbonyl-C₁₋₈-alkyl, C₁₋₈-alkoxycarbonylamino-C₂₋₈-alkoxy, C₁₋₈-alkoxycarbonylamino-C₁₋₈-alkyl, C₁₋₈-alkyl, (N-C₁₋₈-alkyl)-C₁₋₈-alkoxy-C₁₋₈-alkylcarbamoyl, (N-C₁₋₈-alkyl)-C₁₋₈-alkoxy-C₁₋₈-alkylcarbonylamino, (N-C₁₋₈-alkyl)-C₁₋₈-alkoxy-carbonylamino, (N-C₁₈-alkyl)-C₁₈-alkylcarbonylamino-C₂₋₈-alkoxy, (N-C₁₋₈-alkyl)-C₁₋₈-alkylcarbonylamino-C₁₋₈-alkyl, (N-C₁₋₈-alkyl)-C₁₋₈-alkylsulfonylamino-C₂₋₈-alkoxy, (N-C₁₋₈-alkyl)-C₁₋₈-alkylsulfonylamino-C₁₋₈-alkyl, C₁₋₈-alkyl-amidinyl, C₁₋₈-alkylamino-C₂₋₈-alkoxy, di-C₁₋₈-alkylamino-C₂₋₈-alkoxy, C₁₋₈-alkylamino-C₁₋₈-alkyl, di-C₁₋₈-alkylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl, C₁₋₈-alkylaminocarbonylamino-C₂₋₈-alkoxy, C₁₋₈-alkylaminocarbonylamino-C₁₋₈-alkyl, C₁₋₈-alkylcarbonylamino, C₁₋₈-alkylcarbonylamino-C₂₋₈-alkoxy, C₁₋₈-alkylcarbonylamino-C₁₋₈-alkyl, C₁₋₈-alkylcarbonyloxy-C₂₋₈-alkoxy, C₁₋₈-alkylcarbonyloxy-C₁₋₈-alkyl, C₁₋₈-alkylsulfonyl, C₁₋₈-alkylsulfonyl-C₁₋₈-alkoxy, C₁₋₈-alkylsulfonyl-C₁₋₈-alkyl, C₁₋₈-alkylsulfonylamino-C₂₋₈-alkoxy, C₁₋₈-alkylsulfonylamino-C₁₋₈-alkyl, optionally N-mono- oder N,N-di-C₁₋₈-alkylated amino, aryl-C₀₋₈-alkoxy, aryl-C₀₋₈-alkyl, optionally N-mono- oder N,N-di-C₁₋₈-alkylated carbamoyl-C₀₋₈-alkoxy, carboxy-C₁₋₈-alkoxy, carboxy-C₁₋₈-alkoxy-C₁₋₈-alkyl, carboxy-C₁₋₈-alkyl, C₁₋₈-alkyl , cyano-C₂₋₈-alkoxy, cyano-C₁₋₈-alkyl, C₃₋₈-cycloalkyl-C₁₋₈-alkoxy, C₃₋₈-cycloalkyl-carbamoyl-C₀₋₈-alkyl, C₃₋₈-cycloalkylcarbonylamino-C₂₋₈-alkoxy, C₃₋₈-cycloalkylcarbonylamino-C₁₋₈-alkyl, O,N-dimethylhydroxylamino-C₁₋₈-alkyl, halogen, halogen-C₁₋₈-alkoxy, halogen-C₁₋₈-alkyl, heterocyclyl-C₀₋₈-alkoxy, heterocydyl-C₀₋₈-alkyl, heterocyclyl-carbonyl, hydroxy-C₂₋₈-alkoxy-C₁₋₈-alkoxy, hydroxy-C₂₋₈-alkoxy-C₁₋₈-alkyl, hydroxy-C₁₋₈-alkyl, oxide or oxo;
**Q** is
a) , in formula (I), >CR²R³, >CHR⁴ or >CH₂; or
b) , in formula (II), =CH-;
**T** is -Alk-**M**, -Alk-O-**M**, -Alk-O-C(O)-**M**, -Alk-S-**M**, -Alk-NR¹-**M**, -C(O)-NR¹-**M**, -Alk-C(O)-NR¹-**M**, -C(O)-NR¹-Alk-**M**, -Alk-C(O)-NR¹-Alk-**M**, -Alk-NR¹-C(O)-**M**, -Alk-NR¹-C(O)-Alk-**M**, -Alk-S(O)₂-NR¹-**M**, -Alk-S(O)₂-NR¹-Alk-**M**, -Alk-NR¹-S(O)₂-**M** or -Alk-NR¹-S(O)₂-Alk-**M**, wherein Alk represents C₁₋₈-alkylene, which is optionally substituted with halogen;
**V** is C₂₋₈-alkenyloxy, C₂₋₈-alkenyloxy-C₁₋₈-alkyl, C₁₋₈-alkoxy, C₁₋₈-alkoxy-C₁₋₈-alkoxy, optionally halogen-substituted C₁₋₈-alkoxy-C₁₋₈-alkoxy-C₁₋₈-alkoxy, optionally halogen-substituted C₁₋₈-alkoxy-C₁₋₈-alkoxy-C₁₋₈-alkyl , optionally halogen-substituted C₁₋₈-alkoxy-C₁₋₈-alkoxy-C₁₋₈-alkoxy-C₁₋₈-alkyl, C₁₋₈-alkoxy-C₁₋₈-alkyl, C₁₋₈-alkoxy-C₂₋₈-alkylamino-C₁₋₈-al kyl , C₁₋₈-alkoxy-C₀₋₈-alkyl-C₃₋₈-cycloalkyl-C₀₋₈-alkoxy-C₁₋₈-alkyl, C₁₋₈-alkoxy-C₂₋₈-alkylsulfanyl, C₁₋₈-alkoxy-C₂₋₈-alkylsulfanyl-C₁₋₈-alkyl, C₁₋₈-alkoxy-C₃₋₈-cycloalkyl-C₁₋₈-alkoxy, C₁₋₈-alkyl, C₁₋₈-alkyl-sulfanyl-C₂₋₈-alkoxy, C₁₋₈-alkyl-sulfonyl-C₂₋₈-alkoxy, C₁₋₈-alkylsulfanyl-C₂₋₈-alkoxy-C₁₋₈-alkyl, C₁₋₈-alkylsulfanyl-C₁₋₈-alkyl, C₁₋₈-alkylsulfonyl-C₂₋₈-alkoxy-C₁₋₈-alkyl, C₂₋₈-alkynyloxy, C₃₋₈-cycloalkyl-C₀₋₈-alkoxy-C₁₋₈-alkoxy-C₁₋₈-alkyl or C₃₋₈-cycloalkyl-C₀₋₈-alkoxy-C₁₋₈-alkyl, heterocyclyl-C₀₋₈-alkoxy-C₁₋₈-alkoxy-C₀₋₈-alkyl, heterocydyl-sulfanyl-C₁₋₈-alkoxy-C₀₋₈-alkyl, heterocyclyl-C₀₋₈-alkoxy-C₁₋₈-alkyl ;
**W** is aryl or heterocyclyl, especially isoxazolyl, oxadiazolyl, oxazolyl, phenyl, pyrazolyl, pyridyl, pyrimidinyl or thiazolyl, where -in addition to the substituent Veach of said radicals may be optionally substituted with 1-2 C₁₋₈-alkyl, C₁₋₈-alkoxy or halogen;
**R¹** is C₂₋₈-alkenyl, C₁₋₈-alkyl, C₂₋₈-alkynyl, aryl, C₃₋₈-cycloalkyl, C₃₋₈-cycloalkyl-C₁₋₈-alkyl or hydrogen;
**R²** and **R³** are each, independently of the other, C₁-C₈-alkyl or halogen, or, together with the carbon atom to which they are bonded, are a C₃-C₈-cycloalkylidene radical; and
**R⁴** is optionally halogen- and/or hydroxy-substituted C₁₋₈-alkoxy, optionally halogen- and/or hydroxy-substituted C₁₋₈-alkoxy-C₁₋₈-alkoxy, optionally N-mono- or N,N-di-C₁₋₈-alkylated amino-C₂₋₈-alkoxy, optionally N-mono- or N,N-di-C₁₋₈-alkylated or optionally hydroxy-substituted amino-C₀₋₈-alkylcarbonyl-C₁₋₈-alkoxy, hydroxy-C₁₋₈-alkylcarbonyl-C₀₋₈-alkoxy, C₁₋₈-alkoxy-C₀₋₈-alkylcarbonyl-C₀₋₈-alkoxy, optionally N-C₁₋₈-alkylated C₁₋₈-alkoxycarbonylamino-C₂₋₈-alkoxy, optionally N-C₁₋₈-alkylated C₁₋₈-alkoxy-C₂₋₈-alkylaminO-C₂₋₈-alkoxy, optionally N-C₁₋₈-alkylated or optionally halogen-substituted C₁₋₈-alkylcarbonylamino-C₂₋₈-alkoxy, cyano-C₂₋₈-alkoxy, optionally N-C₁₋₈-alkylated or optionally halogen-substituted C₃₋₈alkylcalkyl-C₀₋₈-alkylcarbonylamino-C₂₋₈-alkoxy, optionally N-C₁₋₈-alkylated hydroxy-C₂₋₈-alkylamino-C₂₋₈-alkoxy, heterocyclylcarbonyl-C₀₋₈-al kylamino-C₂-₈-alkoxy, C₁₋₈-alkoxycarbonylamino-C₂₋₈-alkoxy, optionally N-C₁₋₈-alkylated or optionally halogen-substituted heterocyclyl-C₀₋₈-alkylcarbonylamino-C₂₋₈-alkoxy, C₃₋₈-cycloalkyl-C₀₋₈-alkoxy, C₃₋₈-cycloalkyloxy-C₂₋₈-alkoxy, heterocyclyl-C₀₋₈-(optionally hydroxy-substituted)alkoxy, optionally N-C₁₋₈-alkylated heterocyclyl-C₀₋₈-alkylamino-C₀₋₈-alkylcarbonyl-C₀₋₈-alkoxy, C₁₋₈-alkylsulfonyl-C₁₋₈-alkoxy, C₃₋₈-alkynyloxy, heterocyclyl-C₃₋₈-alkynyloxy, optionally N-mono- or N,N-di-C₁₋₈-alkylated amino-C₃₋₈-alkynyloxy, N-mono- or N,N-di-C₁₋₈-alkylated aminocarbonyl-C₃₋₈-alkynyloxy, heterocyclylcarbonyl-C₀₋₈-alkoxy, heterocyclycloxy-C₂₋₈-alkoxy, optionally halogen- and/or hydroxy-substituted C₁₋₈-alkyl, optionally halogen- and/or hydroxy-substituted C₁₋₈-alkoxy-C₁₋₈-alkyl, optionally N-mono- or N,N-di-C₁₋₈-alkylated amino-C₁₋₈-alkyl, optionally N-mono- or N,N-di-C₁₋₈-alkylated or optionally hydroxy-substituted amino-C₀₋₈-alkylcarbonyl-C₁₋₈-alkyl, hydroxy-C₁₋₈-alkylcarbonyl-C₀₋₈-alkyl, C₁₋₈-alkoxy-C₀₋₈-alkylcarbonyl-C₀₋₈-alkyl , optionally N-C₁₋₈-alkylated C₁₋₈-alkoxycarbonylamino-C₁₋₈-alkyl , optionally N-C₁₋₈-alkylated C₁₋₈-alkoxy-C₂₋₈-alkylamino-C₁₋₈-alkyl, optionally N-C₁₋₈-alkylated or optionally halogen-substituted C₁₋₈-alkylcarbonylamino-C₁₋₈-alkyl, cyano-C₁₋₈-alkyl, optionally N-C₁₋₈-alkylated or optionally halogen-substituted C₃₋₈-cycloalkyl-C₀₋₈-alkylcarbonylamino-C₁₋₈-alkyl, optionally N-C₁₋₈-alkylated hydroxy-C₂₋₈-alkylamino-C₁₋₈-alkyl, heterocyclylcarbonyl-C₀₋₈-alkylamino-C₁₋₈-alkyl , C₁₋₈-alkoxycarbonylamino-C₁₋₈-alkyl, optionally N-C₁₋₈-alkylated or optionally halogen-substituted heterocydyl-C₀₋₈-alkylcarbonylamino-C₁₋₈-alkyl, C₃₋₈-cycloalkyl-C₀₋₈-alkyl, C₃₋₈-cycloalkyloxy-C₁₋₈-alkyl, heterocyclyl-C₀₋₈-(optionally hydroxy-substituted)alkyl, optionally N-C₁₋₈-alkylated heterocyclyl-C₀₋₈-alkylamino-C₀₋₈-alkylcarbonyl-C₀₋₈-alkyl, C₁₋₈-alkylsulfonyl-C₁₋₈-alkyl, C₂₋₈-alkynyl, heterocyclyl-C₂8-alkynyl, optionally N-mono- or N,N-di-C₁₋₈-alkylated amino-C₂₋₈-alkynyl, N-mono- or N,N-di-C₁₋₈-alkylated aminocarbonyl-C₂₋₈-alkynyl, heterocyclylcarbonyl-C₀₋₈-alkyl, heterocyclyloxy-C₁₋₈-alkyl, hydroxy or oxo.

Examples of alkyl and alkoxy radicals, which may be linear or branched, are C₁₋₈-alkyl and C₁₋₈-alkoxy radicals such as methyl, ethyl, n-propyl, isopropyl, n-butyl, isobutyl, sec-butyl, tert-butyl, pentyl, hexyl, and methoxy, ethoxy, propoxy, isopropoxy, butoxy, isobutoxy, sec-butoxy and tert-butoxy respectively, in addition, the meaning of "C₀-alkyl" in the above (and hereinafter) mentioned C₀₋₈-alkyl groups is a bond or, if located at a terminal position, a hydrogen atom and the meaning of "C₀-alkoxy" in the above (and hereinafter) mentioned C₀₋₈-alkoxy groups is "-O-" or, if located at a terminal position, an -OH group. C₁₋₈-Alkylenedioxy radicals are preferably methylenedioxy, ethylenedioxy and propylenedioxy. Examples of C₁₋₈-alkanoyl radicals, which may be linear or branched, are acetyl, propionyl and butyryl. Cycloalkyl is a saturated, cyclic hydrocarbon radical having 3-12 carbon atoms, i.e. cyclopropyl, cyclobutyl, cyclopentyl, cyclohexyl, cycloheptyl, bicyclo[2.2.1]heptyl, cyclooctyl, bicyclo[2.2.2]octyl and adamantyl. C₁₋₈-Alkylene radicals, which may be linear or branched, are, for example, methylene, ethylene, 1-methylmethylene, propylene, methylethylene, 1-ethylmethylene, 1,1-dimethylmethylene, 2-methylpropylene, 2-methylbutylene, 2-methylbutyl-2-ene, butyl-2-ene, butyl-3-ene, propyl-2-ene, tetra-, penta- and hexamethylene. C₂₋₈-Alkenylene radicals, which may be linear or branched, are, for example, vinylene and propenylene. C₂₋₈-Alkynylene radicals, which may be linear or branched, are, for example, ethynylene; acyl radicals are alkanoyl radicals, preferably C₁₋₈-alkanoyl radicals, or aroyl radicals such as benzoyl.

Aryl denotes mono- or polycyclic aromatic radicals which may be mono- or polysubstituted, for example phenyl, substituted phenyl, naphthyl or substituted naphthyl. Examples of substituents on such aryl radicals are acetamidinyl-C₁₋₈-alkyl, acyl-C₁₋₈-alkoxy-C₁₋₈-alkyl, (N-acyl)-C₁₋₈-alkoxy-C₁₋₈-alkylamino, C₂₋₈-alkenyl, C₂₋₈-alkenyloxy, C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, (N-C₁₋₈-alkoxy)-C₁₋₈-alkylaminocarbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkyl-carbamoyl, C₁₋₈-alkoxy-C₁₋₈-alkylcarbonyl, C₁₋₈-alkoxy-C₁₋₈-alkyl-carbonylamino, C₁₋₈-alkoxy-C₁₋₈-alkyl-heterocyclyl, 2-C₁₋₈-alkoxy-C₁₋₈-alkyl-4-oxo-imidazol-1-yl, 6-alkoxyaminocarbonyl-C₁₋₈-alkoxy, C₁₋₈-alkoxy-aminocarbonyl-C₁₋₈-alkyl, C₁₋₈-alkoxycarbonyl, C₁₋₈-alkoxycarbonyl-C₁₋₈-alkoxy, C₁₋₈-alkoxycarbonyl-C₁₋₈-alkyl, C₁₋₈-alkoxycarbonylamino-C₁₋₈-alkoxy, C₁₋₈-alkoxycarbonylamino-C₁₋₈-alkyl, C₁₋₈-alkoxycarbonylphenyl, C₁₋₈-alkyl, (N-C₁₋₈-alkyl)-C₁₋₈-alkoxy-C₁₋₈-alkyl-carbamoyl, (N-C₁₋₈-alkyl)-C₁₋₈-alkoxy-C₁₋₈-alkyl-carbonylamino, (N-C₁₋₈-alkyl)-C₁₋₈-alkoxy-carbonylamino, (N-C₁₋₈-alkyl)-C₀₋₈-alkylcarbonylamino-C₁₋₈-alkoxy, (N-C₁₋₈-alkyl)-C₀₋₈-alkylcarbonylamino-C₁₋₈-alkyl, (N-C₁₋₈-alkyl)-C₁₋₈-alkylsulfonylamino-C₁₋₈-alkoxy, (N-C₁₋₈-alkyl)-C₁₋₈-alkylsulfonylamino-C₁₋₈-alkyl, C₁₋₈-alkylamidinyl, optionally N-mono or N,N-di-C₁₋₈-alkylated amino, C₁₋₈-alkylamino-C₂₋₈-alkoxy, di-C₁₋₈-alkylamino-C₂₋₈-alkoxy, C₁₋₈-alkylamino-C₁₋₈-alkyl, di-C₁₋₈-alkylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl, C₁₋₈-alkylaminocarbonylamino-C₁₋₈-alkoxy, C₁₋₈-alkylaminocarbonylamino-C₁₋₈-alkyl, C₁₋₈-alkyl-carbamoyl, di-C₁₋₈-alkylcarbamoyl, C₀₋₈-alkylcarbonylamino, C₀₋₈-alkylcarbonylamino-C₁₋₈-alkoxy, C₀₋₈-alkylcarbonylamino-C₁₋₈-alkyl, C₁₋₈-alkylcarbonyloxy, C₁₋₈-alkylcarbonyloxy-C₁₋₈-alkoxy, C₁₋₈-alkylcarbonyloxy-C₁₋₈-alkyl, C₁₋₈-alkylenedioxy, C₁₋₈-alkyl-sulfonyl, C₁₋₈-alkylsulfonyl-C₁₋₈-alkoxy, C₁₋₈-alkylsulfonyl-C₁₋₈-alkyl, C₁₋₈-alkylsulfonylamino-C₁₋₈-alkoxy, C₁₋₈-alkylsulfonylamino-C₁₋₈-alkyl, aryl-C₁₋₈-alkanoyl, aryl-C₀₋₈-alkoxy, aryl-C₀₈-alkyl, arylamino, aryloxy, arylthio, benzoyloxy-C₁₋₈-alkoxy, benzyloxy, carbamoyl-C₀₈-alkoxy, carbamoyl-C₀₋₈-alkyl, carboxy, carboxy-C₁₋₈-alkoxy, carboxy-C₁₋₈-alkoxy-C₁₈-alkyl, carboxy-C₁₋₈-alkyl, cyano, cyano-C₁₋₈-alkoxy, cyano-C₁₋₈-alkyl, C₃₋₈-cycloalkyl-C₁₋₈-alkanoyl, C₃₋₈-cycloalkylcarbonylamino-C₁₋₈-alkoxy, C₃₋₈-cycloalkylcarbonylamino-C₁₋₈-alkyl, cyclopropyl-C₁₋₈-alkoxy, cyclopropyl-C₁₋₈-alkyl, O,N-dimethylhydroxylamino-C₁₋₈-alkyl, dioxolanyl-C₁₋₈-alkoxy, halogen, halogen-C₁₋₈-alkoxy, halogen-C₁₋₈-alkyl, heterocyclyl, heterocyclyl-C₁₋₈-alkanoyl, heterocyclyl-C₁₋₈-alkoxy, heterocydyl-C₁₋₈-alkoxy-C₁₋₈-alkoxy, heterocydyl-C₁₋₈-alkoxy-C₁₋₈-alkyl, heterocyclyl-C₁₋₈-alkyl, heterocyclylamino, heterocyclyloxy, heterocyclylthio, hydroxy, hydroxy-C₂₋₈-alkoxy, hydroxy-C₂₋₈-alkoxy-C₁₋₈-alkoxy, hydroxy-C₂₋₈-alkoxy-C₁₋₈-alkyl, hydroxy-C₁₋₈-alkyl, (N-hydroxy)-C₁₋₈-alkylaminocarbonyl-C₁₋₈-alkoxy, (N-hydroxy)-C₁₋₈-alkylaminocarbonyl-C₁₋₈-alkyl, hydroxy-C₁₋₈-alkylphenyl, (N-hydroxy)-aminocarbonyl-C₁₋₈alkoxy, (N-hydroxy)-aminocarbonyl-C₁₋₈-alkyl, hydroxybenzyloxy, methylendioxybenzyloxy, methoxybenyloxy, O-methyloximyl-C₁₋₈-alkyl, nitro, 2-oxo-oxazolidinyl-C₁₋₈-alkoxy, 2-oxo-oxazolidinyl-C₁₋₈-alkyl or pyridylcarbonylamino-C₁₋₈-alkyl .

The term "heterocyclyl" denotes mono- or polycyclic, saturated and unsaturated heterocyclic radicals having from 1 to 4 nitrogen and/or 1 or 2 sulfur or oxygen atoms and which may be mono- or polysubstituted, especially mono- ,di- or trisubstituted. Additionally, the term "heterocyclyl" includes the above mentioned oxo-substituted radicals. Examples of unsaturated heterocyclyl radicals are benzo[1,3]dioxolyl, benzofuranyl, benzoimidazolyl, benzooxazolyl, benzothiazolyl, benzo[b]thienyl, quinazolinyl, quinolyl, quinoxalinyl, dihydrobenzofuranyl, 1,3-dihydro-benzoimidazol, 3,4-dihydro-2H-benzo[1,4]oxazinyl, dihydro-3H-benzo[1,4]oxazinyl, 1,4-dihydrobenzo[d][1,3]oxazin, dihydro-2H-benzo[1,4]thiazinyl, 3,4-dihydro-1H-quinazolin, 3,4-dihydro-1H-quinolin, 2,3-dihydroindolyl, dihydro-1H-pyrido[2,3-b][1,4]oxazinyl, 1,1-dioxo-dihydro-2H-benzo[1,4]thiazinyl, furyl, imidazolyl, imidazo[1,5-a]pyridinyl, imidazo[1,2-a]pyrimidinyl, indazolyl, indolyl, isobenzofuranyl, isoquinolyl, isoxazolyl, [1,5]naphthyridyl, oxazolyl, 1-oxido-pyridyl, 2-oxo-benzoimidazolyl, 3-oxo-4H-benzo[1,4]oxazinyl, 2-oxo-benzooxazolyl, 3-oxo-4H-benzo[1,4]thiazinyl, 2-oxo-dihydro-benzo[e][1,4]diazepinyl, 2-oxo-1,3-dihydro-benzoimidazol, 2-oxo-dihydrobenzo-[d][1,3]oxazinyl, 2-oxo-3,4-dihydro-1 H-quinazolin, 2-oxo-3,4-dihydro-1 H-quinolin, 4-oxo-dihydro-imidazolyl, 2-oxo-1,3-dihydroindolyl, 1-oxo-3H-isobenzofuranyl, 2-oxo-1 H-pyrido[2,3-b][1,4]oxazinyl, 2-oxo-1,3,4,5-tetrahydrobenzo[b]azepin, 2-oxo-tetrahydro-benzo[e][1,4]diazepinyl, 4-oxo-3H-thieno[2,3-d]pyrimidinyl, 5-oxo-4H-[1,2,4]triazinyl, phthalazinyl, pyranyl, pyrazinyl, pyrazolyl, pyridyl, pyrimidinyl, 1 H-pyrrolizinyl, pyrrolo[3,2-c]pyridinyl, pyrrolo[2,3-c]pyridinyl, pyrrolo[3,2-b]pyridinyl, 1H-pyrrolo[2,3-b]pyridyl, pyrrolyl, 1,3,4,5-tetrahydrobenzo[b]azepin, tetrahydroquinolinyl, tetrahydroquinoxalinyl, tetrahydroisoquinolinyl, tetrazolyl, thiazolyl, thienyl, triazinyl, triazolyl, 1,1,3-trioxo-dihydro-2H-1lambda*6*-benzo[1,4]thiazinyl, [1,2,3]triazolo[1,5-a]pyridinyl or [1,2,4]triazolo[4,3-a]pyridinyl.

The term "saturated heterocyclyl" denotes 3-16 membered mono- or bicyclic, saturated heterocyclic radicals having from 1 to 4 nitrogen and/or 1 or 2 sulfur or oxygen atoms. Prefered are 3-8 membered, especially preferred 5- or 6-membered monocyclic radicals, which may be condensed to a 3-8 membered, carbocyclic or heterocyclic ring. Another preferred group of saturated heterocyclic radicals are bicyclic radicals possessing a spirocyclic or bridged ring skeleton. Preferred heterocyclic radicals are possessing per ring 1 nitrogen, oxygen or sulfur atom, 1-2 nitrogen atoms and 1-2 oxygen atoms or 1-2 nitrogen atoms and 1-2 sulfur atoms, whereby, per ring, at least 1 carbon atom, preferentially 1-7 carbon atoms are present. Heterocyclyl radicals which comprise a nitrogen atom may be linked either via the N atom or via a C atom to the remainder of the molecule.

Examples for saturated heterocyclyl radicals are azepanyl, azetidinyl, aziridinyl, 3,4-dihydroxypyrrolidinyl, 2,6-dimethylmorpholinyl, 3,5-dimethylmorpholinyl, dioxanyl, [1,4]dioxepanyl, dioxolanyl, 4,4-dioxothiomorpholinyl, dithianyl, dithiolanyl, 2-hydroxymethylpyrrolidinyl, 4-hydroxypiperidinyl, 3-hydroxypyrrolidinyl, 4-methylpiperazinyl, 1-methylpiperidinyl, 1-methylpyrrolidinyl, morpholinyl, oxathianyl, oxazolidinyl, oxepanyl, 2-oxo-azepanyl, 2-oxo-imidazolidinyl, 2-oxo-oxazolidinyl, 2-oxo-piperidinyl, 4-oxo-piperidinyl, 2-oxo-pyrrolidinyl, 2-oxo-tetrahydro-pyrimidinyl, 4-oxo-thiomorpholinyl, piperazinyl, piperidinyl, pyrrolidinyl, tetrahydrofuranyl, tetrahydropyranyl, tetrahydrothiophenyl, tetrahydrothiopyranyl, thiepanyl or thiomorpholinyl.

Examples for saturated bicyclic heterocyclyl radicals are 2,5-dioxabicyclo[4.1.0]heptanyl, 2-oxa-bicyclo[2.2.1]heptanyl, 2-oxa-bicyclo[4.1.0]heptanyl, 3-oxa-bicyclo[4.1.0]heptanyl, 7-oxa-bicyclo[2.2.1]heptanyl, 2-oxa-bicyclo[3.1.0]hexanyl, 3-oxa-bicyclo[3.1.0]hexanyl, 1-oxa-spiro[2.5]octanyl, 6-oxa-spiro[2.5]octanyl or 3-oxa-bicyclo[3.3.1]nonanyl.

Heterocyclyl radicals may be unsubstituted or mono- or polysubstituted, for example mono- or disubstituted. Examples of substituents on such heterocyclyl radicals are C₁₋₆-alkanoyl, C₂₋₆-alkenyl, C₂₋₆-alkinyl, C₁₋₆-alkoxy, C₁₋₆-alkoxy-C₁₋₆-alkoxy, C₁₋₆-al koxy-C₁₋₆-alkyl, C₁₋₆-alkoxycarbonylamino-C₂₋₆-alkoxy, C₁₋₆-alkoxycarbonylamino-C₀₋₆-alkyl, C₁₋₆-alkyl, C₀₋₆-alkylcarbonylamino-C₂₋₆-alkoxy, C₀₋₆-alkylcarbonylamino-C₀₋₆-alkyl, C₁₋₆-alkylcarbonyloxy, C₁₋₆-alkylenedioxy, optionally N-mono or N,N-di-C₁₋₆-alkylated amino, aryl, optionally N-mono or N,N-di-C₁₋₆-alkylated carbamoyl, optionally esterified carboxy, cyano, C₃₋₈-cydoalkoxy, halogen, heteroaryl, heterocyclyl, hydroxy, nitro, oxid, oxo, polyhalogen-C₁₋₆-alkoxy or polyhalogen-C₁₋₆-alkyl.

The term "polyhydroxyalkyl" denotes C₁₋₈-alkyl radicals which may be substituted by 2-8 hydroxyl groups, for example glyceryl, arabityl, sorbityl, etc.
Halogen or halo denotes, for example, fluorine, chlorine or bromine, or a radical singly, multiply or fully substituted by fluorine, chlorine or bromine.
In case of a polysubstituted radical, each individual substituent is selected independently of the other(s).

The compounds of formula (II) have at least one asymmetric carbon atom and may therefore exist in the form of optically pure enantiomers, mixtures of enantiomers or as enantiomeric racemates. The invention encompasses all these forms.

Salts of compounds with salt-forming groups are in particular acid addition salts, salts with bases or, if a plurality of salt-forming groups is present, optionally also mixed salts or inner salts.

Salts are primarily the pharmaceutically acceptable or non-toxic salts of compounds of formulae (I) and (II).

Such salts are formed for example by compounds of the formulae (I) and (II) having an acidic group, e.g. a carboxy or sulfo group, and are for example their salts with suitable bases, such as non-toxic metal salts derived from metals of group la, Ib, IIa and llb of the Periodic Table of the Elements, e.g. alkali metal, in particular lithium, sodium or potassium, salts, alkaline earth metal salts, for example magnesium or calcium salts, furthermore zinc salts or ammonium salts, also salts formed with organic amines such as optionally hydroxy-substituted mono-, di- or trialkylamines, especially mono-, di- or tri-lower-alkylamines, or with quaternary ammonium bases, e.g. methyl-, ethyl-, diethyl- or triethylamine, mono-, bis- or tris(2-hydroxy-lower-alkyl)amines such as ethanol-, diethanol- or triethanolamine, tris(hydroxymethyl)methylamine or 2-hydroxy-tertiary-butylamine, N.N-di-lower-alkyl-N-(hydroxy-lower-alkyl)amine, such as N,N-dimethyl-N-(2-hydroxyethyl)amine, or N-methyl-D-glucamine, or quaternary ammonium hydroxides such as tetrabutylammonium hydroxide. The compounds of the formulae (I) and (II) having a basic group, e.g. an amino group, can form acid addition salts, e.g. with suitable inorganic acids, e.g. hydrohalic acid such as hydrochloric acid, hydrobromic acid, sulfuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulfonic or phosphonic acids or N-substituted sulfamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, furthermore amino acids such as, for example, the α-amino acids mentioned hereinabove, and methanesulfonic acid, ethanesulfonic acid, 2-hydroxyethanesulfonic acid, ethane-1 ,2-disulfonic acid, benzenesulfonic acid, 4-toluenesulfonic acid, naphthalene-2-sulfonic acid, 2- or 3-phosphoglycerate, glucose 6-phosphate, N-cyclohexylsulfamic acid (to form cyclamates) or with other acidic organic compounds such as ascorbic acid. Compounds of formulae (I) and (II) having acidic and basic groups may also form inner salts.

Pharmaceutically unsuitable salts may also be used for isolation and purification.

The groups of compounds mentioned hereinafter are not to be regarded as closed; on the contrary, it is possible for parts of these groups of compounds to be interchanged or replaced by the definitions given above, or omitted, in a worthwhile manner, e.g. to replace general by more specific definitions. The definitions mentioned apply within the scope of general chemical principles such as, for example, the usual valencies of atoms.

The compounds of the formulae (I) and (II) can be prepared in an analogous manner to preparation processes disclosed in the literature. Similar preparation processes are described for example in WO 2004/002957, WO 2004/096769 and WO 2005/040120. Details of the specific preparation variants can be found in the examples.

A preferred group of compounds of formulae (I) and (II) and the salts thereof, preferably the pharmaceutically acceptable salts thereof, are compounds, wherein M is aryl or heterocyclyl, especially phenyl or pyridyl, each of which is is optionally substituted by 1-4 C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkyl or halogen.

A further preferred group of compounds of formulae (I) and (II) and the salts thereof, preferably the pharmaceutically acceptable salts thereof, are compounds in which T is -C(O)-NR¹-Alk-M for formula (I) or -Alk-C(O)-NR'-Alk-M for formula (II), wherein R¹ is preferably C₁₋₈-alkyl or C₃₋₈-cydoalkyl, especially preferably cyclopropyl.

An especially preferred group of compounds of formulae (I) and (II) and the salts thereof, preferably the pharmaceutically acceptable salts thereof, are compounds wherein
M is aryl or heterocyclyl, especially phenyl or pyridyl, each of which is substituted by preferably 1-4 C₁₋₈-alkoxy, C₁₋₈-alkoxy-C₁₋₈-alkoxy, C₁₋₈-alkoxy-C₁₋₈-alkoxy-C₁₋₈-alkyl , C₁₋₈-alkoxy-C₁₋₈-alkyl, C₁₋₈-alkyl or halogen, especially preferably 2-3 C₁₋₈-alkoxy, C₁₋₈-alkyl or halogen;
Q is
a) , in formula (I), >CH₂; or
b) , in formula (II), =CH-;
T is -C(O)-NR¹-Alk-M for formula (I) or -Alk-C(O)-NR¹-Alk-M for formula (II), wherein R¹ is preferably C₁₋₈-alkyl or C₃₋₈-cydoalkyl, especially preferably cyclopropyl, and Alk is methylene;
V is C₁₋₈-alkoxy, C₁₋₈-alkoxy-C₁₋₈-alkoxy, optionally halogen-substituted C₁₋₈-alkoxy-C₁₋₈-alkoxy-C₁₋₈-alkoxy, optionally halogen-substituted C₁₋₈-alkoxy-C₁₋₈-alkoxy-C₁₋₈-alkyl, optionally halogen-substituted C₁₋₈-alkoxy-C₁₋₈-alkoxy-C₁₋₈-alkoxy-C₁₋₈-alkyl, C₁₋₈-alkoxy-C₁₋₈-alkyl, C₁₋₈-alkoxy-C₀₋₈-alkyl-C₃₋₈-cycloalkyl-C₀₋₈-alkoxy-C₁₋₈-alkyl, C₁₋₈-alkoxy-C₂₋₈-alkylsulfanyl, C₁₋₈-alkoxy-C₂₋₈-alkylsulfanyl-C₁₋₈-alkyl, C₁₋₈-alkoxy-C₃₋₈-cycloalkyl-C₁₋₈-alkoxy, C₁₋₈-alkyl, C₁₋₈-alkyl-sulfanyl-C₂₋₈-alkoxy, C₁₋₈-alkylsulfanyl-C₂₋₈-alkoxy-C₁₋₈-alkyl, C₁₋₈-alkylsulfanyl-C₁₋₈-alkyl, C₁₋₈-alkylsulfonyl-C₂₋₈-alkoxy-C₁₋₈-alkyl, C₃₋₈-cycloalkyl-C₀₋₈-alkoxy-C₁₋₈-alkoxy-C₁₋₈-alkyl or C₃₋₈-cycloalkyl-C₀₋₈-alkoxy-C₁₋₈-alkyl , heterocyclyl-C₀₋₈-alkoxy-C₁₋₈-alkoxy-C₀₋₈-alkyl, heterocyclyl-sulfanyl-C₁₋₈-alkoxy-C₀₋₈-alkyl, heterocyclyl-C₀₋₈-alkoxy-C₁₋₈-alkyl; and
W is aryl or heterocyclyl, especially isoxazolyl, oxadiazolyl, oxazolyl, phenyl or thiazolyl.

The compounds of formulae (I) and (II) can also be prepared in optically pure form. Separation into antipodes can take place by methods known per se, either preferably at an early stage in the synthesis by salt formation with an optically active acid such as, for example, (+)- or (-)-mandelic acid and separation of the diastereomeric salts by fractional crystallization or preferably at a rather late stage by derivatizing with a chiral auxiliary component such as, for example, (+)- or (-)-camphanoyl chloride, and separation of the diastereomeric products by chromatography and/or crystallization and subsequent cleavage of the linkage to the chiral auxiliary. The pure diastereomeric salts and derivatives can be analysed to determine the absolute configuration of the contained piperidine by conventional spectroscopic methods, with X-ray spectroscopy on single crystals representing a particularly suitable method.

The compounds of formulae (I) and (II) also include compounds in which one or more atoms are replaced by their stable, non-radioactive isotopes; for example a hydrogen atom by deuterium.

Prodrug derivatives of the compounds described herein are derivatives thereof which on *in vivo* use liberate the original compound by a chemical or physiological process. A prodrug may for example be converted into the original compound when a physiological pH is reached or by enzymatic conversion. Possible examples of prodrug derivatives are esters of freely available carboxylic acids, S- and O-acyl derivatives of thiols, alcohols or phenols, the acyl group being defined as above. Preferred derivatives are pharmaceutically acceptable ester derivatives which are converted by solvolysis in physiological medium into the original carboxylic acid, such as, for example, lower alkyl esters, cycloalkyl esters, lower alkenyl esters, benzyl esters, mono- or disubstituted lower alkyl esters such as lower ω-(amino, mono- or dialkylamino, carboxy, lower alkoxycarbonyl) - alkyl esters or such as lower α-(alkanoyloxy, alkoxycarbonyl or dialkylaminocarbonyl) - alkyl esters; conventionally, pivaloyloxymethyl esters and similar esters are used as such.

Because of the close relationship between a free compound, a prodrug derivative and a salt compound, a particular compound in this invention also includes its prodrug derivative and salt form, where this is possible and appropriate.

The compounds of formulae (I) and (II) and their pharmaceutically acceptable salts have an inhibitory effect on the natural enzyme renin. The latter passes from the kidneys into the blood and there brings about the cleavage of angiotensinogen to form the decapeptide angiotensin I which is then cleaved in the lung, the kidneys and other organs to the octapeptide angiotensin II. Angiotensin II raises the blood pressure both directly by arterial constriction, and indirectly by releasing the hormone aldosterone, which retains sodium ions, from the adrenals, which is associated with an increase in the extracellular fluid volume. This increase is attributable to the effect of angiotensin II itself or of the heptapeptide angiotensin III formed therefrom as cleavage product. Inhibitors of the enzymatic activity of renin bring about a reduction in the formation of angiotensin I and, as a consequence thereof, the formation of a smaller amount of angiotensin II. The reduced concentration of this active peptide hormone is the direct cause of the blood pressure-lowering effect of renin inhibitors.

The effect of renin inhibitors is detected inter alia experimentally by means of in vitro tests where the reduction in the formation of angiotensin I is measured in various systems (human plasma, purified human renin together with synthetic or natural renin substrate). The following in vitro test of Nussberger et al. (1987) J. Cardiovascular Pharmacol., Vol. 9, pp. 39-44, is used inter alia. This test measures the formation of angiotensin I in human plasma. The amount of angiotensin I formed is determined in a subsequent radioimmunoassay. The effect of inhibitors on the formation of angiotensin I is tested in this system by adding various concentrations of these substances. The IC₅₀ is defined as the concentration of the particular inhibitor which reduces the formation of angiotensin I by 50%. The compounds of the present invention show inhibitory effects in the in vitro systems at minimal concentrations of about 10⁻⁶ to about 10⁻¹⁰ mol/l.

Renin inhibitors bring about a fall in blood pressure in salt-depleted animals. Human renin differs from renin of other species. Inhibitors of human renin are tested using primates (marmosets, Callithrix jacchus) because human renin and primate renin are substantially homologous in the enzymatically active region. The following in vivo test is employed inter alia: the test compounds are tested on normotensive marmosets of both sexes with a body weight of about 350 g, which are conscious, unrestrained and in their normal cages. Blood pressure and heart rate are measured with a catheter in the descending aorta and are recorded radiometrically. Endogenous release of renin is stimulated by combining a low-salt diet for 1 week with a single intramuscular injection of furosemide (5-(aminosulfonyl)-4-chloro-2-[(2-furanylmethyl)amino]benzoic acid) (5 mg/kg). 16 hours after the furosemide injection, the test substances are administered either directly into the femoral artery by means of a hypodermic needle or as suspension or solution by gavage into the stomach, and their effect on blood pressure and heart rate is evaluated. The compounds of the present invention have a blood pressure-lowering effect in the described in vivo test with i.v. doses of about 0.003 to about 0.3 mg/kg and with oral doses of about 0.3 to about 30 mg/kg.

The blood pressure-reducing effect of the compounds described herein can be tested *in vivo* using the following protocol:
The investigations take place in 5 to 6-week old, male double transgenic rats (dTGR), which overexpress both human angiotensinogen and human renin and consequently develop hypertension (Bohlender J. et al., J. Am. Soc. Nephrol. 2000; 11: 2056-2061). This double transgenic rat strain was produced by crossbreeding two transgenic strains, one for human angiotensinogen with the endogenous promoter and one for human renin with the endogenous promoter. Neither single transgenic strain was hypertensive. The double transgenic rats, both males and females, develop severe hypertension (mean systolic pressure, approximately 200 mm Hg) and die after a median of 55 days if untreated. The fact that human renin can be studied in the rat is a unique feature of this model. Age-matched Sprague-Dawley rats serve as non-hypertensive control animals. The animals are divided into treatment groups and receive test substance or vehicle (control) for various treatment durations. The applied doses for oral administration may range from 0.5 to 100 mg/kg body weight. Throughout the study, the animals receive standard feed and tap water ad libitum. The systolic and diastolic blood pressure, and the heart rate are measured telemetrically by means of transducers implanted in the abdominal aorta, allowing the animals free and unrestricted movement.

The effect of the compounds described herein on kidney damage (proteinuria) can be tested *in vivo* using the following protocol:
The investigations take place in 4-week old, male double transgenic rats (dTGR), as described above. The animals are divided into treatment groups and receive test substance or vehicle (control) each day for 7 weeks. The applied doses for oral administration may range from 0.5 to 100 mg/kg body weight. Throughout the study, the animals receive standard feed and tap water ad libitum. The animals are placed periodically in metabolism cages in order to determine the 24-hour urinary excretion of albumin, diuresis, natriuresis, and urine osmolality. At the end of the study, the animals are sacrificed and the kidneys and hearts may also be removed for determining the weight and for immunohistological investigations (fibrosis, macrophage/T cell infiltration, etc.).

The pharmacokinetic properties of the compounds described herein can be tested *in vivo* using the following protocol:
The investigations take place in pre-catheterized (carotid artery) male rats (300 g ± 20%) that can move freely throughout the study. The compound is administered intravenously and orally (gavage) in separate sets of animals. The applied doses for oral administration may range from 0.5 to 50 mg/kg body weight; the doses for intravenous administration may range from 0.5 to 20 mg/kg body weight. Blood samples are collected through the catheter before compound administration and over the subsequent 24-hour period using an automated sampling device (AccuSampler, DiLab Europe, Lund, Sweden). Plasma levels of the compound are determined using a validated LC-MS analytical method. The pharmacokinetic analysis is performed on the plasma concentration-time curves after averaging all plasma concentrations across time points for each route of administration. Typical pharmacokinetics parameters to be calculated include: maximum concentration (Cₘₐₓ), time to maximum concentration (tₘₐₓ), area under the curve from 0 hours to the time point of the last quantifiable concentration (AUC₀₋ₜ), area under the curve from time 0 to infinity (AUC_{0-inf}), elimination rate constant (K), terminal half-life (t_{½}), absolute oral bioavailability or fraction absorbed (F), clearance (CL), and Volume of distribution during the terminal phase (Vd).

The compounds of formulae (I) and (II) and their pharmaceutically acceptable salts can be used as medicines, e.g. in the form of pharmaceutical products. The pharmaceutical products can be administered enterally, such as orally, e.g. in the form of tablets, lacquered tablets, sugar-coated tablets, hard and soft gelatine capsules, solutions, emulsions or suspensions, nasally, e.g. in the form of nasal sprays, rectally, e.g. in the form of suppositories, or transdermally, e.g. in the form of ointments or patches. However, administration is also possible parenterally, such as intramuscularly or intravenously, e.g. in the form of solutions for injection.

Tablets, lacquered tablets, sugar-coated tablets and hard gelatine capsules can be produced by processing the compounds of the formulae (I) and (II) and their pharmaceutically acceptable salts with pharmaceutically inert inorganic or organic excipients. Excipients of these types which can be used for example for tablets, sugar-coated tablets and hard gelatine capsules are lactose, maize starch or derivatives thereof, talc, stearic acid or salts thereof etc.

Excipients suitable for soft gelatine capsules are, for example, vegetable oils, waxes, fats, semisolid and liquid polyols etc.

Excipients suitable for producing solutions and syrups are, for example, water, polyols, sucrose, invert sugar, glucose etc.

Excipients suitable for solutions for injection are, for example, water, alcohols, polyols, glycerol, vegetable oils, bile acids, lecithin etc.

Excipients suitable for suppositories are, for example, natural or hardened oils, waxes, fats, semiliquid or liquid polyols etc.

The pharmaceutical products may in addition comprise preservatives, solubilizers, viscosity-increasing substances, stabilizers, wetting agents, emulsifiers, sweeteners, colorants, aromatizers, salts to alter the osmotic pressure, buffers, coating agents or antioxidants. They may also comprise other substances of therapeutic value.

The present invention further provides the use of the compounds of the formulae (I) and (II) and their pharmaceutically acceptable salts in the treatment or prevention of high blood pressure, heart failure, glaucoma, myocardial infarction, renal failure, restenoses and stroke.

The compounds of formulae (I) and (II) and their pharmaceutically acceptable salts can also be administered in combination with one or more agents having cardiovascular activity, e.g. α- and β-blockers such as phentolamine, phenoxybenzamine, prazosin, terazosin, tolazine, atenolol, metoprolol, nadolol, propranolol, timolol, carteolol etc.; vasodilators such as hydralazine, minoxidil, diazoxide, nitroprusside, flosequinan etc.; calcium antagonists such as amrinone, bencyclan, diltiazem, fendiline, flunarizine, nicardipine, nimodipine, perhexiline, verapamil, gallopamil, nifedipine etc.; ACE inhibitors such as cilazapril, captopril, enalapril, lisinopril etc.; potassium activators such as pinacidil; antiserotoninergics such as ketanserine; thromboxane synthetase inhibitors; neutral endopeptidase inhibitors (NEP inhibitors); angiotensin II antagonists; and diuretics such as hydrochlorothiazide, chlorothiazide, acetazolamide, amiloride, bumetanide, benzthiazide, ethacrynic acid, furosemide, indacrinone, metolazone, spironolactone, triamterene, chlorthalidone etc.; sympatholytics such as methyldopa, clonidine, guanabenz, reserpine; and other agents suitable for the treatment of high blood pressure, heart failure or vascular disorders associated with diabetes or renal disorders such as acute or chronic renal failure in humans and animals. Such combinations can be used separately or in products which comprise a plurality of components.

Further substances which can be used in combination with the compounds of formulae (I) and (II) are the compounds of classes (i) to (ix) on page 1 of WO 02/40007 (and the preferences and examples detailed further therein) and the substances mentioned on pages 20 and 21 of WO 03/027091.

The dosage may vary within wide limits and must of course be adapted to the individual circumstances in each individual case. In general, a daily dose appropriate for oral administration ought to be from about 3 mg to about 3 g, preferably about 10 mg to about 1 g, e.g. approximately 300 mg per adult person (70 kg), divided into preferably 1-3 single doses, which may be for example of equal size, although the stated upper limit may also be exceeded if this proves to be indicated, and children usually receive a reduced dose appropriate for their age and body weight.

### EXAMPLES

The following examples illustrate the present invention. All temperatures are stated in degrees Celsius and pressures in mbar. Unless mentioned otherwise, the reactions take place at room temperature. The abbreviation "Rf = xx (A)" means for example that the Rf is found in solvent system A to be xx. The ratio of amounts of solvents to one another is always stated in parts by volume. Chemical names for final products and intermediates have been generated with the aid of the AutoNom 2000 (Automatic Nomenclature) program.

The following abbreviations are used:
Rf ratio of distance migrated by a substance to the distance of the solvent front from the starting point in thin-layer chromatography

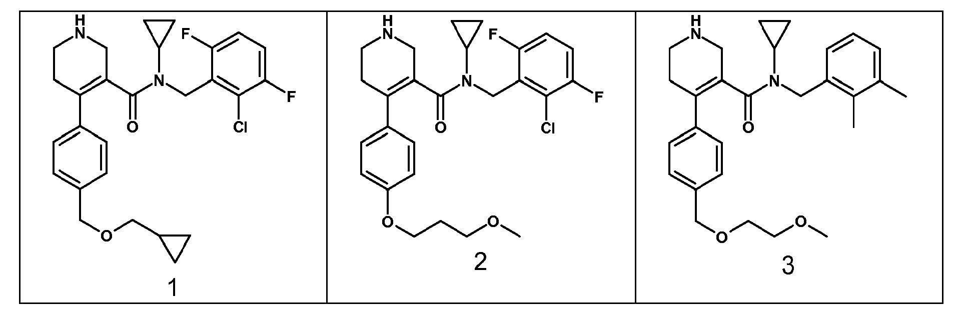

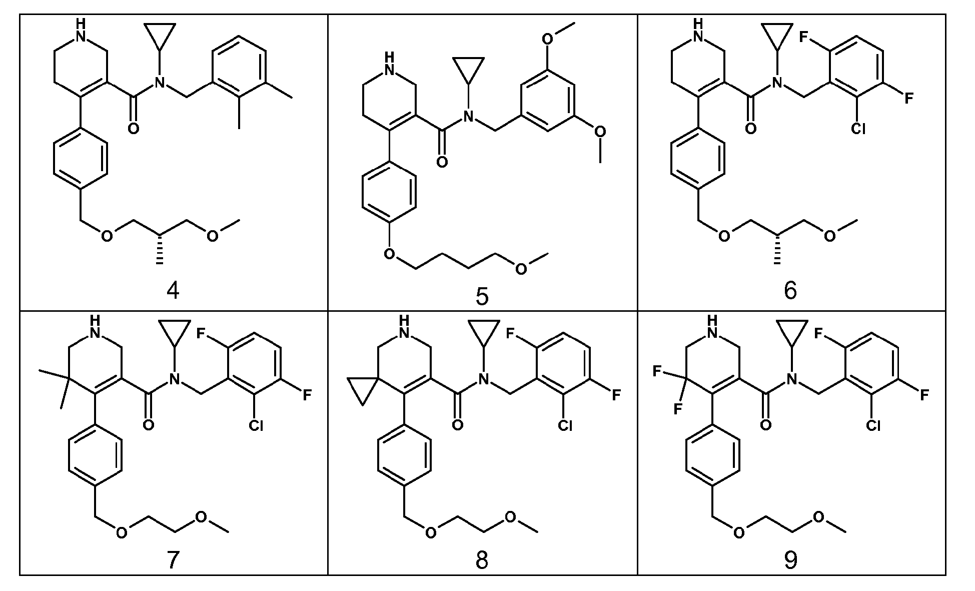

### General Method A: (N-BOC deprotection)

10 mmol of HCl (4M in dioxane) are added to a solution of 1 mmol "N-BOC derivative" in 10 ml of dichloromethane at 0°C, and the mixture is then stirred for 2 hours at 0°C and 2 hours at room temperature. The reaction mixture is then evaporated. The title compound is obtained from the residue by flash chromatography (SiO₂ 60F).

### General Method B: (phenol alkylation)

A mixture of 20 mmol of "phenol" in 60 ml of N,N-dimethylformamide with 30 mmol of potassium carbonate and 30 mmol of "halide" or "tosylate" is stirred at 80-100°C for 12-24 hours. The reaction mixture is then evaporated. The residue is diluted with 1 M aqueous sodium bicarbonate solution and extracted with ethyl acetate (3X). The combined organic phases are washed with brine, dried with sodium sulfate and evaporated. The title compound is obtained from the residue by flash chromatography (SiO₂ 60F).

### General Method C: (O-alkylation)

1.1 mmol of sodium hydride (60% dispersion in oil) are added to a solution of 1 mmol of "alcohol" and 1.0-2.0 mmol of "benzyl halide" in 2.0 ml of N,N-dimethylformamide while stirring at -10°C. The reaction mixture is stirred at -10°C for 1 hour and at room temperature for 18 hours. The mixture is poured into 1 M aqueous sodium bicarbonate solution and extracted with tert-butyl methyl ether (2X). The combined organic phases are washed successively with water and brine, dried with sodium sulfate and evaporated. The title compound is obtained from the residue by flash chromatography (SiO₂ 60F).

### General Method D: (chlorination)

12 mmol of methanesulfonyl chloride are slowly added to a mixture of 10 mmol of "benzyl alcohol", 16 mmol of triethylamine and 1 mmol of tetrabutylammonium chloride in 150 ml of dichloromethane at 0°C. The reaction mixture is stirred at 0°C for 1 hour and at room temperature for 18 hours. The mixture is diluted with 1:1 water/brine and extracted with dichloromethane (2X). The combined organic phases are washed successively with 1 M aqueous sodium bicarbonate solution and brine, dried with sodium sulfate and evaporated. The title compound is obtained from the residue by flash chromatography (SiO₂ 60F).

### General Method E: (alcohol desilylation)

A solution of 1 mmol of "silyl ether" in 5 ml of tetrahydrofuran is mixed with 1.5-2.0 mmol of tetrabutylammonium fluoride (1 M solution in tetrahydrofuran) and the solution is stirred at room temperature for 1-2 hours. The reaction solution is then diluted with water and extracted with tert-butyl methyl ether (2X). The combined organic phases are dried with sodium sulfate and evaporated. The title compound is obtained from the residue by flash chromatography (SiO₂ 60F).

### General procedure F: (amide-formation I)

To a stirred solution of 1.0 mmol of "acid" and "1.0 mmol of "amin" in 20 ml of dichloromethane are added 5.0 mmol of triethylamin and 1.0 mmol of tri-propylphosphonic acid cyclic anhydride [68957-94-8] (50% in ethyl acetate) at room temperature. The reaction mixture is stirred for 1-3 hours, diluted with dichloromethane, washed with 1N HCl and brine. The organic phase is dried over sodium sulfate and the solvent is concentrated under reduced pressure. The residue is purified by flash chromatography (SiO₂ 60F) to afford the title compound.

### General Method G: (amide-formation II)

1.2-1.8 mmol of (1-chloro-2-methylpropenyl)dimethylamine (chloro-enamine) are added to a solution of 1 mmol of "acid" in 10 ml of dichloromethane at 0°C. After 2-4 hours, the reaction mixture is evaporated and the residue is dissolved in 6 ml of dichloromethane - this solution is added dropwise to the solution of 1.25 mmol of "amine" and 1.1 mmol of triethylamine in 6 ml of dichloromethane at 0°C over the course of 2-10 minutes. The reaction mixture is stirred at room temperature for 1-2 hours, poured into water and extracted with tert-butyl methyl ether (2X). The combined organic phases are washed with brine, dried with sodium sulfate and evaporated. The title compound is obtained from the residue by flash chromatography (SiO₂ 60F).

### Example 1

### 4-(4-Cyclopropylmethoxymethyl-phenyl)-1,2,5,6-tetrahydro-pyridine-3-carboxylic acid (2-chloro-3,6-difluoro-benzyl)-cyclopropyl-amide

According to general method A, 5-[(2-chloro-3,6-difluoro-benzyl)-cyclopropylcarbamoyl]-4-(4-cyclopropylmethoxymethyl-phenyl)-3,6-dihydro-2H-pyridine-1-carboxylic acid tert-butyl ester is used to afford the title compound which is identified based on its Rf value.

The starting material(s) is(are) prepared as follows:
a) 5-[(2-Chloro-3,6-difluoro-benzyl)-cyclopropyl-carbamoyl]-4-(4-cyclopropylmethoxymethyl-phenyl)-3,6-dihydro-2H-pyridine-1-carboxylic acid tert-butyl ester
   According to general method G, 4-(4-cyclopropylmethoxymethyl-phenyl)-5,6-dihydro-2H-pyridine-1,3-dicarboxylic acid 1-tert-butyl ester and (2-chloro-3,6-difluoro-benzyl)-cyclopropyl-amine [625437-41-4] are used to afford the title compound which is identified based on its Rf value.
b) 4-(4-Cyclopropylmethoxymethyl-phenyl)-5,6-dihydro-2H-pyridine-1,3-dicarboxylic acid 1-tert-butyl ester
   A solution of 0.35 mmol of 4-(4-cyclopropylmethoxymethyl-phenyl)-5,6-dihydro-2H-pyridine-1,3-dicarboxylic acid 1-tert-butyl ester 3-methyl ester in 3.5 ml of methanol is treated with 3.5 ml of 1 M NaOH, and the mixture is stirred at 70°C for 5 hours. The reaction mixture is cooled to room temperature and partitioned between tert-butylmethyl ether and a 1:1 mixture of 1 M HCl and brine. The organic layer is washed with brine - the combined aqueous layers are extracted with tert-butylmethyl ether (2X). The combined organic layers are dried with sodium sulfate and evaporated to provide the crude title compound which is identified based on its Rf value.
c) 4-(4-Cyclopropylmethoxymethyl-phenyl)-5,6-dihydro-2H-pyridine-1,3-dicarboxylic acid 1-tert-butyl ester 3-methyl ester
   A solution of 2.36 mmol of 1-bromo-4-cyclopropylmethoxymethyl-benzene in 8 ml of tetrahydrofuran at -78°C is treated with 2.64 mmol of n-butyllithium (1.6M/hexane). After stirring for 1 hour, 2.90 mmol of zinc chloride (1 M/tetrahydrofuran) is added and the cooling bath is removed. After 1.5 hours, a solution of 0.94 mmol of 4-trifluoromethanesulfonyloxy-5,6-dihydro-2H-pyridine-1,3-dicarboxylic acid 1-tert-butyl ester 3-methyl ester [161491-25-4] in 4 ml of tetrahydrofuran is added, followed by a solution of 0.05 mmol of tetrakis(triphenylphosphine)palladium(0) in 1 ml of tetrahydrofuran, and the resulting mixture is warmed to 50°C for 45 minutes. The reaction is then cooled to room temperature and partitioned between ethyl acetate and saturated aqueous ammonium chloride solution. The aqueous layer is extracted with ethyl acetate (2X) - the combined organic layers are dried with sodium sulfate and evaporated. The residue is purified by flash chromatography (SiO₂ 60F) to afford the title compound which is identified based on its Rf value.
d) 1-Bromo-4-cyclopropylmethoxymethyl-benzene
   According to general method C, 1-bromo-4-chloromethyl-benzene [589-17-3] and cyclopropyl-methanol [2516-33-8] are used to afford the title compound which is identified based on its Rf value.

According to the procedures described in example 1, the following compounds are prepared in an analogous manner:
2 4-[4-(3-Methoxy-propoxy)-phenyl]-1,2,5,6-tetrahydro-pyridine-3-carboxylic acid (2-chloro-3,6-difluoro-benzyl)-cyclopropyl-amide
   starting from 1-bromo-4-(3-methoxy-propoxy)-benzene [279262-34-9]
3 4-[4-(2-Methoxy-ethoxymethyl)-phenyl]-1,2,5,6-tetrahydro-pyridine-3-carboxylic acid cyclopropyl-(2,3-dimethyl-benzyl)-amide
   starting from 1-bromo-4-(2-methoxy-ethoxymethyl)-benzene [166959-29-1] and using cyclopropyl-(2,3-dimethyl-benzyl)-amine [625437-38-9] instead of (2-chloro-3,6-difluoro-benzyl)-cyclopropyl-amine [625437-41-4] in step a.
4 4-[4-((S)-3-Methoxy-2-methyl-propoxymethyl)-phenyl]-1,2,5,6-tetrahydropyridine-3-carboxylic acid cyclopropyl-(2,3-dimethyl-benzyl)-amide
   starting from 1-bromo-4-((S)-3-methoxy-2-methyl-propoxymethyl)-benzene and using cyclopropyl-(2,3-dimethyl-benzyl)-amine [625437-38-9] instead of (2-chloro-3,6-difluoro-benzyl)-cyclopropyl-amine [625437-41-4] in step a.

The starting material(s) is(are) prepared as follows:
a) 1-Bromo-4-((S)-3-methoxy-2-methyl-propoxymethyl)-benzene
   According to general method C, 1-bromo-4-chloromethyl-benzene [589-17-3] and (R)-3-methoxy-2-methyl-propan-1-ol (Example 3a) are used to afford the title compound which is identified based on its Rf value.
5 4-[4-(4-Methoxy-butoxy)-phenyl]-1,2,5,6-tetrahydro-pyridine-3-carboxylic acid cyclopropyl-(3,5-dimethoxy-benzyl)-amide
   starting from 1-bromo-4-(4-methoxy-butoxy)-benzene [303976-49-0] and using cyclopropyl-(3,5-dimethoxy-benzyl)-amine [625435-20-3] instead of (2-chloro-3,6-difluoro-benzyl)-cyclopropyl-amine [625437-41-4] in step a.
6 4-[4-((S)-3-Methoxy-2-methyl-propoxymethyl)-phenyl]-1,2,5,6-tetrahydropyridine-3-carboxylic acid (2-chloro-3,6-difluoro-benzyl)-cyclopropyl-amide
   starting from 1-bromo-4-((S)-3-methoxy-2-methyl-propoxymethyl)-benzene (Example 4a)
7 4-[4-(2-Methoxy-ethoxymethyl)-phenyl]-5,5-dimethyl- 1,2,5,6-tetrahydropyridine-3-carboxylic acid (2-chloro-3,6-difluoro-benzyl)-cycloprolpyl-amide
   starting from 1-bromo-4-(2-methoxy-ethoxymethyl)-benzene [166959-29-1] and using 5,5-dimethyl-4-trifluoromethanesulfonyloxy-5,6-dihydro-2H-pyridine-1,3-dicarboxylic acid 1-tert-butyl ester 3-methyl ester [851314-40-4] instead of 4-trifluoromethanesulfonyloxy-5,6-dihydro-2H-pyridine-1,3-dicarboxylic acid 1-tert-butyl ester 3-methyl ester [161491-25-4] in step c.
8 8-[4-(2-Methoxy-ethoxymethyl)-phenyl]-5-aza-sipiro[2.5]oct-7-ene-7-carboxylic acid (2-chloro-3,6-difluoro-benzyl)-cyclopropyl-amide
   starting from 1-bromo-4-(2-methoxy-ethoxymethyl)-benzene [166959-29-1] and using 8-trifluoromethanesulfonyloxy-5-aza-spiro[2.5]oct-7-ene-5,7-dicarboxylic acid 5-tert-butyl ester 7-methyl ester [851314-38-0] instead of 4-trifluoromethanesulfonyloxy-5,6-dihydro-2H-pyridine-1,3-dicarboxylic acid 1-tert-butyl ester 3-methyl ester [161491-25-4] in step c.

### Example 9

### 5,5-Difluoro-4-[4-(2-methoxy-ethoxymethyl)-phenyl]-1,2,5,6-tetrahydro-ipyridine-3-carboxylic acid (2-chloro-3,6-difluoro-benzyl)-cycloprolpyl-amide

According to general method A, 5-[(2-chloro-3,6-difluoro-benzyl)-cyclopropyl-carbamoyl]-3,3-difluoro-4-[4-(2-methoxy-ethoxymethyl)-phenyl]-3,6-dihydro-2H-pyridine-1-carboxylic acid tert-butyl ester is used to afford the title compound which is identified based on its Rf value.

The starting material(s) is(are) prepared as follows:
a) 5-[(2-Chloro-3,6-difluoro-benzyl)-cyclopropyl-carbamoyl]-3,3-difluoro-4-[4-(2-methoxy-ethoxymethyl)-phenyl]-3,6-dihydro-2H-pyridine-1-carboxylic acid tert-butyl ester
   According to general method G, 5,5-difluoro-4-[4-(2-methoxy-ethoxymethyl)-phenyl]-5,6-dihydro-2H-pyridine-1,3-dicarboxylic acid 1-tert-butyl ester and (2-chloro-3,6-difluoro-benzyl)-cyclopropyl-amine [625437-41-4] are used to afford the title compound which is identified based on its Rf value.
b) 5,5-Difluoro-4-[4-(2-methoxy-ethoxymethyl)-phenyl]-5,6-dihydro-2H-pyridine-1,3-dicarboxylic acid 1-tert-butyl ester
   Analogously to Example 1 b, 5,5-difluoro-4-[4-(2-methoxy-ethoxymethyl)-phenyl]-5,6-dihydro-2H-pyridine-1,3-dicarboxylic acid 1-tert-butyl ester 3-ethyl ester is used to afford the title compound which is identified based on its Rf value.
c) 5,5-Difluoro-4-[4-(2-methoxy-ethoxymethyl)-phenyl]-5,6-dihydro-2H-pyridine-1,3-dicarboxylic acid 1-tert-butyl ester 3-ethyl ester
   A mixture of 2.1 mmol of 1-benzyl-5,5-difluoro-4-[4-(2-methoxy-ethoxymethyl)-phenyl]-1,2,5,6-tetrahydro-pyridine-3-carboxylic acid ethyl ester, 2.3 mmol of di-tert-butyl dicarbonate and 0.1 g of 10% Pd/C in 10 ml of ethanol is hydrogenated at room temperature for 24 hours. The reaction mixture is filtered and concentrated under reduced pressure. The residue is purified by flash chromatography (SiO₂ 60F) to afford the title compound which is identified based on its Rf value.
d) 1-Benzyl-5,5-difluoro-4-[4-(2-methoxy-ethoxymethyl)-phenyl]-1,2,5,6-tetrahydropyridine-3-carboxylic acid ethyl ester
   Analogously to Example 1c, 1-bromo-4-(2-methoxy-ethoxymethyl)-benzene [166959-29-1] and 1-benzyl-5,5-difluoro-4-trifluoromethanesulfonyloxy-1,2,5,6-tetrahydropyridine-3-carboxylic acid ethyl ester [851314-41-5] are used to afford the title compound which is identified based on its Rf value.

## Claims

1. A compound of the general formula (I) or (II) 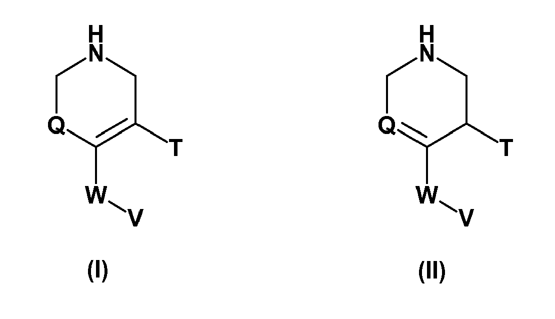 and its salt or compound in which one or more atoms are replaced by their stable, non-radioactive isotopes, especially its pharmaceutically acceptable salt, wherein
**M** (as part of T) is aryl or heterocyclyl, especially benzoimidazolyl, benzo[1,3]dioxolyl, benzofuranyl, benzooxazolyl, benzothiazolyl, benzo[b]thienyl, 2H-chromenyl, 3,4-dihydro-2H-benzo[1,4]oxazinyl, dihydro-3H-benzo[1,4]oxazinyl, dihydro-2H-benzo[1,4]thiazinyl, 1 a,7b-dihydro-1 H-cyclopropa[c]chromenyl, 2,3-dihydro-1 H-indolyl, dihydro-1H-pyrido[2,3-b][1,4]oxazinyl, imidazo[1,2-a]pyridyl, imidazo[1,5-a]pyridyl, indazolyl, indolyl, isobenzofuranyl, isoquinolyl, [1,5]naphthyridyl, phenyl, phthalazinyl, pyridyl, pyrimidinyl, 1 H-pyrrolo[2,3-b]pyridyl, 1 H-pyrrolo[2,3-c]pyridyl, 1H-pyrrolo[3,2-b]pyridyl, quinazolinyl, quinolyl, quinoxalinyl, tetrahydroimidazo[1,2-a]pyridyl, 1,1 a,2,7b-tetrahydro-cyclopropa[c]chromenyl, tetrahydroimidazo[1,5-a]pyridyl, tetrahydroisoquinolyl, tetrahydroquinolyl, tetrahydroquinoxalinyl, [1,2,3]triazolo[1,5-a]pyridyl or [1,2,4]triazolo[4,3-a]pyridyl, each of which is optionally substituted by 1-4 acyl-C₁₋₈-alkoxy-C₁₋₈-alkoxy, acyl-C₁₋₈-alkoxy-C₁₋₈-alkyl, (N-acyl)-C₁₋₈-alkoxy-C₁₋₈-alkylamino, C₁₋₈-alkanoyl, C₁₋₈-alkoxy, C₁₋₈-alkoxy-C₁₋₈-alkanoyl, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, (N-C₁₋₈-alkoxy)-C₁₋₈-alkylaminocarbonyl-C₁₋₈-alkoxy, (N-C₁₋₈-alkoxy)-C₁₋₈-alkylaminocarbonyl-C₁₋₈-alkyl, C₁₋₈-alkoxy-C₁₋₈-alkyl-carbamoyl, C₁₋₈-alkoxy-C₁₋₈-alkyl-carbonylamino, C₁₈-alkoxy-C₁₋₈-alkyl-heterocydyl, C₁₋₈-alkoxy-carbonyl, C₁₋₈-alkoxycarbonyl-C₁₋₈-alkoxy, C₁₋₈-alkoxycarbonyl-C₁₋₈-alkyl, C₁₋₈-alkoxycarbonylamino-C₂₋₈-alkoxy, C₁₋₈-alkoxycarbonylamino-C₁₋₈-alkyl, C₁₋₈-alkyl, (N-C₁₋₈-alkyl)-C₁₋₈-alkoxy-C₁₋₈-alkyl-carbamoyl, (N-C₁₋₈-alkyl)-C₁₋₈-alkoxy-C₁₋₈-alkylcarbonylamino, (N-C₁₋₈-alkyl)-C₁₋₈-alkoxycarbonylamino, (N-C₁₋₈-alkyl)-C₁₋₈-alkylcarbonylamino-C₂₋₈-alkoxy, (N-C₁₋₈-alkyl)-C₁₋₈-alkylcarbonylamino-C₁₋₈-alkyl, (N-C₁₋₈-alkyl)-C₁₋₈-alkylsulfonylamino-C₂₋₈-alkoxy, (N-C₁₋₈-alkyl)-C₁₋₈-alkylsulfonylamino-C₁₋₈-alkyl, C₁₋₈-alkyl-amidinyl, C₁₋₈-alkylamino-C₂₋₈-alkoxy, di-C₁₋₈-alkylamino-C₂₋₈-alkoxy, C₁₋₈-alkylamino-C₁₋₈-alkyl, di-C₁₋₈-alkylamino-C₁₋₈-alkyl, C₁₋₈-alkylaminocarbonyl-C₁₋₈-alkoxy, di-C₁₋₈-alkylaminocarbonyl-C₁₋₈-alkoxy, C₁₋₈-alkylaminocarbonyl-C₁₋₈-alkoxy-C₁₋₈-alkyl, C₁₋₈-alkylaminocarbonyl-C₁₋₈-alkyl, di-C₁₋₈-alkylaminocarbonyl-C₁₋₈-alkyl, C₁₋₈-alkylaminocarbonylamino-C₂₋₈-alkoxy, C₁₋₈-alkylaminocarbonylamino-C₁₋₈-alkyl, C₁₋₈-alkylcarbonylamino, C₁₋₈-alkylcarbonylamino-C₂₋₈-alkoxy, C₁₋₈-alkylcarbonylamino-C₁₋₈-alkyl, C₁₋₈-alkyl-carbonyloxy-C₂₋₈-alkoxy, C₁₋₈-alkylcarbonyloxy-C₁₋₈-alkyl, C₁₋₈-alkylsulfonyl, C₁₋₈-alkylsulfonyl-C₁₋₈-alkoxy, C₁₋₈-alkylsulfonyl-C₁₋₈-alkyl, C₁₋₈-alkylsulfonylamino-C₂₋₈-alkoxy, C₁₋₈-alkylsulfonylamino-C₁₋₈-alkyl, optionally N-mono- oder N,N-di-C₁₋₈-alkylated amino, aryl-C₀₋₈-alkoxy, aryl-C₀₋₈-alkyl, optionally N-mono- oder N,N-di-C₁₋₈-alkylated carbamoyl-C₀₋₈-alkoxy, carboxy-C₁₋₈-alkoxy, carboxy-C₁₋₈-alkoxy-C₁₋₈-alkyl, carboxy-C₁₋₈-alkyl, C₁₋₈-alkyl , cyano-C₂₋₈-alkoxy, cyano-C₁₋₈-alkyl, C₃₋₈-cycloalkyl-C₁₋₈-alkoxy, C₃₋₈-cycloalkyl-carbamoyl-C₀₋₈-alkyl, C₃₋₈-cycloalkylcarbonylamino-C₂₋₈-alkoxy, C₃₋₈-cyloalkylcarbonylamino-C₁₋₈-alkyl, O,N-dimethylhydroxylamino-C₁₋₈-alkyl, halogen, halogen-C₁₋₈-alkoxy, halogen-C₁₋₈-alkyl, heterocyclyl-C₀₋₈-alkoxy, heterocyclyl-C₀₋₈-alkyl, heterocyclyl-carbonyl, hydroxy-C₂₋₈-alkoxy-C₁₋₈-alkoxy, hydroxy-C₂₋₈-alkoxy-C₁₋₈-alkyl, hydroxy-C₁₋₈-alkyl, oxide or oxo;
**Q** is
a) , in formula (I), >C**R²R³**, >CH**R⁴** or >CH₂; or
b) , in formula (II), =CH-;
**T** is -Alk-**M**, -Alk-O-**M**, -Alk-O-C(O)-**M**, -Alk-S-**M**, -Alk-N**R¹-M**, -C(O)-N**R¹-M**, -Alk-C(O)-N**R¹**-**M**, -C(O)-NR¹-Alk-**M**, -Alk-C(O)-N**R¹**-Alk-**M**, -Alk-N**R¹**-C(O)-**M**, -Alk-N**R¹-**C(O)-Alk-**M**, -Alk-S(O)₂-NR¹-**M**, -Alk-S(O)₂-N**R¹**-Alk-**M**, -Alk-N**R¹**-S(O)₂-**M** or -Alk-N**R¹-**S(O)₂-Alk-**M**, wherein Alk represents C₁₋₈-alkylene, which is optionally substituted with halogen;
**V** is C₂₋₈-alkenyloxy, C₂₋₈-alkenyloxy-C₁₋₈-alkyl, C₁₋₈-alkoxy, C₁₋₈-alkoxy-C₁₋₈-alkoxy, optionally halogen-substituted C₁₋₈-alkoxy-C₁₋₈-alkoxy-C₁₋₈-alkoxy, optionally halogen-substituted C₁₋₈-alkoxy-C₁₋₈-alkoxy-C₁₋₈-al kyl , optionally halogen-substituted C₁₋₈-alkoxy-C₁₋₈-alkoxy-C₁₋₈-alkoxy-C₁₋₈-alkyl , C₁₋₈-alkoxy-C₁₋₈-alkyl, C₁₋₈-alkoxy-C₂₋₈-alkylamino-C₁₋₈-alkyl, C₁₋₈-alkoxy-C₀₋₈-alkyl-C₃₋₈-cycloalkyl-C₀₋₈-alkoxy-C₁₋₈-alkyl, C₁₋₈-alkoxy-C₂₋₈-alkylsulfanyl, C₁₋₈-alkoxy-C₂₋₈-alkylsulfanyl-C₁₋₈-alkyl, C₁₋₈-alkoxy-C₃₋₈-cycloalkyl-C₁₋₈-alkoxy, C₁₋₈-alkyl, C₁₋₈-alkyl-sulfanyl-C₂₋₈-alkoxy, C₁₋₈-alkyl-sulfonyl-C₂₋₈-alkoxy, C₁₋₈-alkylsulfanyl-C₂₋₈-alkoxy-C₁₋₈-alkyl, C₁₋₈-alkylsulfanyl-C₁₋₈-alkyl, C₁₋₈-alkylsulfonyl-C₂₋₈-alkoxy-C₁₋₈-alkyl, C₂₋₈-alkynyloxy, C₃₋₈-cycloalkyl-C₀₋₈-alkoxy-C₁₋₈-alkoxy-C₁₋₈-alkyl or C₃₋₈-cycloalkyl-C₀₋₈-alkoxy-C₁₋₈-alkyl, heterocyclyl-C₀₋₈-alkoxy-C₁₋₈-alkoxy-C₀₋₈-alkyl, heterocydyl-sulfanyl-C₁₋₈-alkoxy-C₀₋₈-alkyl, heterocyclyl-C₀₋₈-alkoxy-C₁₋₈-alkyl ;
**W** is aryl or heterocyclyl, especially isoxazolyl, oxadiazolyl, oxazolyl, phenyl, pyrazolyl, pyridyl, pyrimidinyl or thiazolyl, where -in addition to the substituent Veach of said radicals may be optionally substituted with 1-2 C₁₋₈-alkyl, C₁₋₈-alkoxy or halogen;
**R¹** is C₂₋₈-alkenyl, C₁₋₈-alkyl, C₂₋₈-alkynyl, aryl, C₃₋₈-cycloalkyl, C₃₋₈-cydoalkyl-C₁₋₈-alkyl or hydrogen;
**R²** and **R³** are each, independently of the other, C₁-C₈-alkyl or halogen, or, together with the carbon atom to which they are bonded, are a C₃-C₈-cydoalkylidene radical; and
**R⁴** is optionally halogen- and/or hydroxy-substituted C₁₋₈-alkoxy, optionally halogen- and/or hydroxy-substituted C₁₋₈-alkoxy-C₁₋₈-alkoxy, optionally N-mono- or N,N-di-C₁₋₈-alkylated amino-C₂₋₈-alkoxy, optionally N-mono- or N,N-di-C₁₋₈-alkylated or optionally hydroxy-substituted amino-C₀₋₈-alkylcarbonyl-C₁₋₈-alkoxy, hydroxy-C₁₋₈-alkylcarbonyl-C₀₋₈-alkoxy, C₁₋₈-alkoxy-C₀₋₈-alkylcarbonyl-C₀₋₈-alkoxy, optionally N-C₁₋₈-alkylated C₁₈-alkoxycarbonylamino-C₂₋₈-alkoxy, optionally N-C₁₋₈-alkylated C₁₋₈-alkoxy-C₂₋₈-alkylamino-C₂₋₈-alkoxy, optionally N-C₁₋₈-alkylated or optionally halogen-substituted C₁₋₈-alkylcarbonylamino-C₂₋₈-alkoxy, cyano-C₂₋₈-alkoxy, optionally N-C₁₋₈-alkylated or optionally halogen-substituted C₃₋₈-cycloalkyl C₀₋₈-alkylcarbonylamino-C₂₋₈-alkoxy, optionally N-C₁₋₈-alkylated hydroxy-C₂₋₈-alkylamino-C₂₋₈-alkoxy, heterocyclylcarbonyl-C₀₋₈-alkylamino-C₂₋₈-alkoxy, C₁₋₈-alkoxycarbonylamino-C₂₋₈-alkoxy, optionally N-C₁₋₈-alkylated or optionally halogen-substituted heterocyclyl-C₀₋₈-alkylcarbonylamino-C₂₋₈-alkoxy, C₃₋₈-cycloalkyl-C₀₋₈-alkoxy, C₃₋₈-cycloalkyloxy-C₂₋₈-alkoxy, heterocyclyl-C₀₋₈-(optionally hydroxy-substituted)alkoxy, optionally N-C₁₋₈-alkylated heterocyclyl-C₀₋₈-alkylamino-C₀₋₈-alkylcarbonyl-C₀₋₈-alkoxy, C₁₋₈-alkylsulfonyl-C₁₋₈-alkoxy, C₃₋₈-alkynyloxy, heterocyclyl-C₃₋₈-alkynyloxy, optionally N-mono- or N,N-di-C₁₋₈-alkylated amino-C₃₋₈-alkynyloxy, N-mono- or N,N-di-C₁₋₈-alkylated aminocarbonyl-C₃₋₈-alkynyloxy, heterocyclylcarbonyl-C₀₋₈-alkoxy, heterocyclyloxy-C₂₋₈-alkoxy, optionally halogen- and/or hydroxy-substituted C₁₋₈-alkyl, optionally halogen- and/or hydroxy-substituted C₁₋₈-alkoxy-C₁₋₈-alkyl, optionally N-mono- or N,N-di-C₁₋₈-alkylated amino-C₁₋₈-alkyl, optionally N-mono- or N,N-di-C₁₋₈-alkylated or optionally hydroxy-substituted amino-C₀₋₈-alkylcarbonyl-C₁₋₈-alkyl, hydroxy-C₁₋₈-alkylcarbonyl-C₀₋₈-alkyl, C₁₋₈-alkoxy-C₀₋₈-alkylcarbonyl-C₀₋₈-alkyl , optionally N-C₁₋₈-alkylated C₁₋₈-alkoxycarbonylamino-C₁₋₈-alkyl , optionally N-C₁₋₈-alkylated C₁₋₈-alkoxy-C₂₋₈-alkylamino-C₁₋₈-alkyl, optionally N-C₁₋₈-alkylated or optionally halogen-substituted C₁₋₈-alkylcarbonylamino-C₁₋₈-alkyl, cyano-C₁₋₈-alkyl, optionally N-C₁₋₈-alkylated or optionally halogen-substituted C₃₋₈-cycloalkyl-C₀₋₈-alkylcarbonylamino-C₁₋₈-alkyl, optionally N-C₁₋₈-alkylated hydroxy-C₂₋₈-alkylamino-C₁₋₈-alkyl, heterocyclylcarbonyl-C₀₋₈-alkylamino-C₁₋₈-al kyl , C₁₋₈-alkoxycarbonylamino-C₁₋₈-alkyl, optionally N-C₁₋₈-alkylated or optionally halogen-substituted heterocydyl-C₀₋₈-alkylcarbonylamino-C₁₋₈-alkyl, C₃₋₈-cydoalkyl-C₀₋₈-alkyl, C₃₋₈-cycloalkyloxy-C₁₋₈-alkyl, heterocyclyl-C₀₋₈-(optionally hydroxy-substituted)alkyl, optionally N-C₁₋₈-alkylated heterocyclyl-C₀₋₈-alkylamino-C₀₈-alkylcarbonyl-C₀₋₈-alkyl, C₁₋₈-alkylsulfonyl-C₁₋₈-alkyl, C₂₋₈-alkynyl, heterocydyl-C₂₋₈-alkynyl, optionally N-mono- or N,N-di-C₁₋₈-alkylated amino-C₂₋₈-alkynyl, N-mono- or N,N-di-C₁₋₈-alkylated aminocarbonyl-C₂₋₈-alkynyl, heterocyclylcarbonyl-C₀₋₈-alkyl, heterocyclyloxy-C₁₋₈-alkyl, hydroxy or oxo.

2. A compound according to claim 1, wherein
**M** is phenyl or pyridyl, each of which is is optionally substituted by 1-4 C₁₋₈-alkoxy, C₁₈-alkoxy-C₁₋₈-alkoxy, C₁₋₈-alkoxy-C₁₋₈-alkoxy-C₁₋₈-al kyl , C₁₋₈-alkoxy-C₁₋₈-al kyl , C₁₋₈-alkyl or halogen.

3. A compound according to either claim 1 or claim 2, wherein
**T** is -C(O)-N**R¹**-Alk-**M** for formula (I) or -Alk-C(O)-N**R¹**-Alk-**M** for formula (II), wherein **R¹** is preferably C₁₋₈-alkyl or C₃₋₈-cycloalkyl, especially preferably cyclopropyl.

4. A compound according to claim 1, wherein
**M** is aryl or heterocyclyl, especially phenyl or pyridyl, each of which is substituted by preferably 1-4 C₁₋₈-alkoxy, C₁₋₈-alkoxy-C₁₋₈-alkoxy, C₁₋₈-alkoxy-C₁₋₈-alkoxy-C₁₋₈-alkyl, C₁₋₈-alkoxy-C₁₋₈-alkyl, C₁₋₈-alkyl or halogen, especially preferably 2-3 C₁₋₈-alkoxy, C₁₋₈-alkyl or halogen;
**Q** is
a) , in formula (1), >CH₂; or
b) , in formula (II), =CH-;
**T** is -C(O)-N**R¹**-Alk-**M** for formula (I) or -Alk-C(O)-N**R¹**-Alk-**M** for formula (II), wherein **R¹** is preferably C₁₋₈-alkyl or C₃₋₈-cydoalkyl, especially preferably cyclopropyl, and Alk is methylene;
**V** is C₁₋₈-alkoxy, C₁₋₈-alkoxy-C₁₋₈-alkoxy, optionally halogen-substituted C₁₋₈-alkoxy-C₁₋₈-alkoxy-C₁₋₈-alkyl, C₁₋₈-alkoxy-C₁₋₈-alkyl, C₁₋₈-alkoxy-C₀₋₈-alkyl-C₃₋₈-cycloalkyl-C₀₋₈-alkoxy-C₁₋₈-alkyl, C₁₋₈-alkoxy-C₂₋₈-alkylsulfanyl, C₁₋₈-alkoxy-C₂₋₈-alkylsulfanyl-C₁₋₈-alkyl, C₁₋₈-alkoxy-C₃₋₈-cycloalkyl-C₁₋₈-alkoxy, C₁₋₈-alkyl, C₁₋₈-alkyl-sulfanyl-C₂₋₈-alkoxy, C₁₋₈-alkylsulfanyl-C₂₋₈-alkoxy-C₁₋₈-alkyl, C₁₋₈-alkylsulfanyl-C₁₋₈-alkyl, C₁₋₈-alkylsulfonyl-C₂₋₈-alkoxy-C₁₋₈-alkyl, C₃₋₈-cycloalkyl-C₀₋₈-alkoxy-C₀₋₈-alkoxy-C₁₋₈-alkyl or C₃₋₈-cycloalkyl-C₀₋₈-alkoxy-C₁₋₈-alkyl ; and
**W** is aryl or heterocyclyl, especially isoxazolyl, oxadiazolyl, oxazolyl, phenyl or thiazolyl .

5. Use of a compound according to any one of claims 1 to 4 for the preparation of a medication, in particular a medication for the treatment or prevention of high blood pressure, heart failure, glaucoma, myocardial infarction, renal failure, restenoses and stroke.

6. A pharmaceutical composition comprising a compound according to any one of claims 1 to 4 and optionally one or more agents having cardiovascular activity.
